# EUROPEAN PATENT APPLICATION

(11) **EP 1 256 626 A1**
(43) Date of publication of application: **13.11.2002**
(21) Application number: 01901529.6
(22) Date of filing: 24.01.2001
(51) Int. Cl.: C12N 15/10, C07H 21/04, G01N 33/50

(54) **SUPPORT FOR FIXING NUCLEOTIDE AND PROCESS FOR PRODUCING THE SAME**

(30) Priority: 27.01.2000 JP 2000019301
(71) Applicant: Toyo Kohan Co., Ltd., Tokyo 102-8447 (JP)
(72) Inventor: TANGA, Michifumi, c/o TOYO KOHAN CO., LTD., Kudamatsu-shi, Yamaguchi 744-8611 (JP); OKAMURA, Hiroshi, c/o TOYO KOHAN CO., LTD., Kudamatsu-shi, Yamaguchi 744-8611 (JP); TAKAGI, Ken-Ichi, c/o TOYO KOHAN CO., LTD., Kudamatsu-shi, Yamaguchi 744-8611 (JP); TAKAHASHI, Kojiro, Hiroshima-shi, Hiroshima 734-0015 (JP)
(74) Representative: VOSSIUS & PARTNER
(86) International application number: JP0100443
(87) International publication number: WO01055365

(57) **Abstract**

A support for fixing a nucleotide which contributes to the efficient clarification of DNA without damaging the terminal parts of the DNA and, therefore, is useful in the fields of biology, biochemistry and the like. This support for fixing a nucleotide is a chemically modified substrate on which oligonucleotide are immobilized characterized in that the oligonucleotide have a restriction enzyme cleavage site. This support is produced by chemically modifying the substrate, immobilizing a single-stranded oligonucleotide thereon, then hybridizing the single-stranded oligonucleotide with another single-stranded oligonucleotide having a base sequence complementary thereto, and ligating an oligonucleotide having a restriction enzyme site at the end thereof.

SEQUENCE LISTING

<110>TOYO KOHAN CO., LTD.

<120>SUPPORT FOR FIXING NUCLEOTIDE AND PROCESS FOR PRODUCING

THE SAME

<130>1738PCT

<150>JP 2000-019301

<151>2000-01-27

<160>2

<210>1

<211>24

<212>DNA

<213>Artifical sequence

<400>1

aaaggttttt tttttttttt tttg 24

<210>2

<211>27

<212>DNA

<213>artifical sequence

<400>2

aattcaaaaa aaaaaaaaaa aaacctt 27

## Description

### Technical Field

The present invention relates to a support for fixing a nucleotide being able to fix nucleic acid, etc. useful in the field of molecular biology and the field related to genetic engineering and also to a process for producing the same.

### Background of the Invention

Gene analysis is useful in the fields of molecular biology and genetic engineering and, in recent years, it has been utilized in the medical field such as finding of diseases as well.

In gene analysis, DNA chips have been developed in recent years and its analyzing velocity has become significantly quick. However, in the conventional DNA chips, there is used a method where a high-molecular substance such as polylysine is applied on the surface of a slide glass or a silicone substrate and, after that, DNA is fixed. There is another method where oligonucleotide is synthesized on a glass substrate using a semiconductor technique such as photolithograph.

However, in the method where DNA is fixed by applying a high-molecular substance such as polylysine on the surface of a slide glass or a silicone substrate, the fixed state of DNA is unstable and there is a problem that DNA is detached during a hybrid forming step or a washing step. Further, in DNA chips using a semiconductor technique, there is a problem of very high cost because of complexity of the manufacturing steps.

In order to solve such problems, it is necessary that DNA is fixed on the surface of a solid substrate in a high density and also in a strong manner.

A substrate where the surface of a solid substrate is chemically modified has been known as well. However, when the desired DNA is directly bonded to the chemically modified part by an amide bond, there is a risk that the terminal part of DNA is damaged by the treatment, etc. during the chemical bonding and, therefore, there has been a demand for an improvement therefor.

An object of the present invention is to provide a support for fixing nucleotide useful in the fields of molecular biology, genetic engineering, etc. whereby clarification of DNA can be efficiently carried out without damaging the terminal part of DNA.

### Disclosure of the Invention

The present inventors have found that, when an oligonucleotide having a predetermined restriction enzyme cleavage site is fixed to a chemically modified substrate so that the restriction enzyme site corresponding to the DNA which is to be fixed is formed, it is now possible to fix in a stable manner whereupon the present invention has been achieved.

Thus, a support for fixing a nucleotide according to the present invention is characterized in that it is a chemically modified substrate to which an oligonucleotide is fixed and the oligonucleotide has a restriction enzyme cleavage site.

In that case, it is preferred that only one of the strands of the oligonucleotide is bonded to the chemically modified substrate and, further, it is preferred that the oligonucleotide is bonded to the chemically modified substrate via an amide bond.

A process for the production of the support for fixing a nucleotide according to the present invention is characterized in that the substrate is chemically modified, a single-stranded oligonucleotide is fixed thereto and then another single-stranded oligonucleotide having a complementary base sequence to the above single-stranded oligonucleotide is hybridized to the said above single-stranded oligonucleotide whereupon the oligonucleotide having a restriction enzyme site at the terminal is bonded.

In that case, it is preferred that the chemical modification of the substrate comprises chlorination, amination and carboxylation of the substrate surface and it is also preferred that, after the substrate is chemically modified, the terminal is activated in the presence of a dehydrating condensing agent. It is further preferred that the dehydrating condensing agent is carbodiimide.

In that case, it is also preferred that the chemical modification of the substrate is that the substrate surface is chlorinated and aminated and that the resulting primary amino group is subjected to a dehydrating condensation with one of ester groups of the activated diester and it is further preferred that the ester group of the activated diester is N-hydroxysuccinimide or p-hydroxysuccinimide.

In that case, it is also preferred that the single-stranded oligonucleotide fixed to the substrate has 1-10 nucleotide(s) having a primary amine at the terminal of the side to be fixed. It is further preferred that the single-stranded oligonucleotide fixed to the substrate has 1-10 primary amine(s) and then 1-5 thymine or guanine at the terminal of the side to be fixed. It is furthermore preferred that the single-stranded oligonucleotide fixed to the substrate has 1-5 adenine or cytosine and then 1-5 thymine or cytosine at the terminal of the side to be fixed.

### Best Mode for Carrying Out the Invention

The support for fixing of a nucleotide according to the present invention is characterized in that it is a substrate where an oligonucleotide is fixed by a chemical modification and the oligonucleotide has a restriction enzyme cleavage site.

A restriction enzyme cleavage site means a sequence of nucleic acid after being specifically cleaved by a restriction enzyme. With regard to the restriction enzyme, there is no particular limitation so far as it is a commonly used one. Its examples are AatI, AatIIAccI, AflII, AluI, Alw44I, ApaI, AseI, AvaI, BamHI, BanI, BanII, BanIII, BbrPI, BclI, BfrI, BglI, BglII, BsiWI, BsmI, BssHII, BstEII, BstXI, Cfr9I, Cfr10I, Cfrl3I, CspI, Csp45I, DdeI, DraI, Eco47I, Eco47III, Eco52I, Eco81I, Eco105I, EcoRI, EcoRII, EcoRV, EcoT22I, EheI, FspI, HaeII, HaeIII, HhaI, Hin1I, HincII, HindIII, HinfI, HpaI, HpaII, KpnI, MboII, MluI, MroI, MscI, MspI, MvaI, NaeI, NarI, NciI, NcoI, NheI, NotI, NruI, NspV, PacI, PpuMI, PstI, PvuI, PvuII, RsaI, SacI, SacII, SalI, Sau3AI, Sau96I, ScaI, ScrFI, SfiI, SmaI, SpeI, SphI, SrfI, SspI, TaqI, TspEI, XbaI and XhoI and a cleavage site for a restriction enzyme which is freely selected from the above is called a restriction enzyme cleavage site.

Since the oligonucleotide has a necessity of having a restriction enzyme cleavage site as such, it is inevitably a double-stranded nucleic acid such as DNA.

With regard to the oligonucleotide, there may be exemplified natural ones such as higher animals, fungi, bacteria and viruses and those which are artificially subjected to a structural change and synthesized and, since it is easily synthesized as will be mentioned later, such a one is preferred. Incidentally, the base numbers of the oligonucleotide are preferably 10-50.

The support for fixing a nucleotide according to the present invention may be easily prepared by the following methods.

### (1) Chemical modification of substrate

In the support for fixing a nucleotide according to the present invention, such an oligonucleotide is fixed to the chemically modified substrate.

Examples of the substrate are glass; diamond; metal such as gold, silver, copper, aluminum, tungsten and molybdenum; a layered product of ceramics with the above-mentioned glass, diamond or metal; and plastics such as polycarbonate and fluorine resin.

Other materials may be also used so far as they are chemically stable materials and there may be exemplified graphite and diamond-like carbon. It is also possible to use a mixture or a layered product of the plastic with the above-mentioned metal, glass, ceramics, diamond, etc.

Among the above, it is preferred to use diamond itself or a thing where diamond is partially used as a substrate in view of thermal conductance. Diamond has an excellent thermal conductance and is able to follow quick heating and cooling whereby it can effectively shorten a heat cycle time where heating and cooling are repeated such as in the case of PCR.

It is preferred that the thermal conductivity of the substrate of the present invention is not less than 0.1 W/cm · K, preferably not less than 0.5 W/cm · K or, particularly preferably, not less than 1.0 W/cm · K. That is because, when it is not less than 1.0 W/cm · K, a follow-up property for heating and cooling is excellent when PCR or the like is carried out after DNA is fixed to the chemically modified part of the support of present invention.

With regard to a material for a diamond substrate, any of synthetic diamond, diamond prepared under high pressure and natural diamond may be used. The structure may be either single crystal or polycrystal. From the viewpoint of productivity, it is preferred to use diamond which is manufactured by a gas-phase synthetic method such as a microwave plasma CVD ( Chemical Vapor deposit ).

Method for formation of a substrate where diamond or other matter is a material may be carried out by a known method. There may be exemplified microwave plasma CVD method, ECR CVD ( Electric Cyclotron Resonance ( Chemical Vapor Deposit) method, IPC ( Inductively Coupled Plasma) method, direct current sputtering method, ECR ( Electric Cyclotron Resonance) sputtering method, ion plating method, arc ion plating method, EB ( Electron Beam) vapor deposition method and resistance heating vapor deposition method.

Further, a product which is bonded and formed by mixing metal powder or ceramic powder with resin as a binder may be exemplified as a method for the manufacture of a substrate. Furthermore, a method where a material such as metal powder or ceramic powder is compressed using a press molding machine and the product is sintered at high temperature may be exemplified as a method for the manufacture of a substrate.

It is preferred that the surface of a substrate is intentionally made rough. This is because, in such a roughened surface, surface area of the substrate increases whereby it is convenient for fixing large amount of DNA, etc. Shape of the substrate may be any of plate, threadlike, spherical, polygonal, discoid, powdery, etc. Further, the substrate may be a complex of diamond with other substance (such as a two-phase substance).

Chemical modification is carried out by substituting the substrate surface with a hydrocarbon group having a polar group such as hydroxyl group, carboxyl group, epoxy group, amino group, thiol group or isocyanate group at the terminal so that the polynucleotide is made to be able to bonded. It is preferred that carbon number(s) of the hydrocarbon moiety of such a hydrocarbon group is/are 0-12 or, more preferably, 0-6. The examples are monocarboxylic acid such as formic acid, acetic acid and propionic acid; dicarboxylic acid such as oxalic acid, malonic acid, succinic acid, maleic acid and fumaric acid; polycarboxylic acid such as trimellitic acid; etc. and it is possible to use in a form of one or more kind(s) of acid anhydride. Oxalic acid and succinic acid are particularly preferred.

It is preferred that the hydrocarbon group is subjected to an amino bond to the substrate surface. This is because, as a result of an amino bond, chemical modification can be made easy and strong.

Such a chemical modification can be achieved in such a manner that the substrate surface is irradiated with ultraviolet ray to chlorinate, irradiated with ultraviolet ray in ammonia gas to aminate and then carboxylated using an appropriate acid chloride or dicarboxylic acid anhydride.

### (2) Fixing a single-stranded oligonucleotide

After that, a single-stranded oligonucleotide (hereinafter, referred to as oligonucleotide A) is fixed to the chemically modified part by an amide bond. The fixation is easily carried out when the terminal of the hydrocarbon group of the chemical modification is activated before the fixation and, therefore, that is preferred. With regard to a dehydrating condensing agent, the use of carbodiimide is particularly preferred.

Besides such methods, it is also possible to form by such a manner that the substrate surface is made into a chlorinated and aminated state (not carboxylated) and the resulting primary amino group is subjected to a dehydrating condensation with one of ester groups of activated diester having ester groups such as N-hydroxysuccinimide and p-nitrophenol.

Although the sequence of the oligonucleotide A is free, it is necessary that the terminal which is not fixed (3'-terminal) is designed in such a manner that a double-stranded oligonucleotide obtained by hybridization with the next and other oligonucleotide has a restriction enzyme cleavage site.

It is also preferred that, by taking the easiness of the amide bond to the chemically modified substrate into consideration, the side to be fixed has 1-10 base(s) of nucleotide having primary amine such as adenine, cytosine and guanine. Particularly preferably, 1-5 base(s) of the side to be fixed (5'-terminal) is/are adenine or cytosine. It is also preferred that 1-5 base(s) after adenine is/are thymine or guanine so as to suppress the divergence in the next hybridization.

### (3) Another single-stranded oligonucleotide is hybridized to oligonucleotide A.

It is necessary that another single-stranded oligonucleotide (hereinafter, referred to as oligonucleotide B) has a complementary sequence to the oligonucleotide A.

It is necessary in the present invention that, in a double-stranded oligonucleotide obtained by hybridization, the terminal which is opposite to the side to be fixed to the substrate has a restriction enzyme cleavage site and, therefore, it is necessary that an oligonucleotide A and an oligonucleotide B form restriction enzyme cleavage sites. Conditions for hybridization may be set as same as that which is usually done.

After a restriction enzyme cleavage site corresponding to the DNA to be conjugated is formed, the support for fixing a nucleotide according to the present invention prepared as such is able to conjugate thereto.

The present invention will now be illustrated in detail by way of the following Examples.

### Example 1

### (Preparation of a support for fixing of a nucleotide having EcoRI cleavage site)

### (1) Chemical modification of the substrate

A substrate comprising diamond was irradiated with ultraviolet ray in chlorine gas so that the surface was chlorinated. After that, it was irradiated with ultraviolet ray in ammonia gas to aminate and then carboxylated by refluxing in chloroform using an acid chloride whereupon the substrate surface was chemically modified.

### (2) Fixing of a single-stranded oligonucleotide

The chemically modified substrate prepared in the above (1) was dipped for 15 minutes in a 1,4-dioxane solution (in an amount of 100 µl to 1 of the diamond substrate) in which 2.5 mg/ml of carbodiimide and 1.5 mg/ml of N-hydroxysuccinimide were dissolved so that the terminal carboxyl group was dehydrated and condensed. After completion of the reaction, the product was washed with water and further washed with a 1,4-dioxane solution followed by drying.

Further, adenine of 5'-terminal of an oligonucleotide A-1 having the sequence mentioned in SEQ ID NO: 1 of the Sequence Listing was subjected to an amide bonding to the activated part.

### (3) Hybridization of an oligonucleotide B to an oligonucleotide A

An oligonucleotide B (sequence = aattcaaaaa aaaaaaaaaa aaacctt: refer to SEQ ID NO: 2 of the Sequence Listing) having a complementary sequence to an oligonucleotide A (sequence = aaaggttttt tttttttttt tttg: refer to SEQ ID NO: 1) was prepared and was hybridized to the oligonucleotide A which was fixed in the above (2).

An oligonucleotide B was dissolved in 78 µl of aseptic water and 20 µl of 20 × ssc (saline-sodium citration solution) and 2 µl of 10% SDS solution were added thereto to make the total amount 100 µl. A substrate to which the oligonucleotide A was fixed was dipped in this solution at 35°C for 10 hours.

As a result, a support for fixing according to the present invention having EcoRI cleavage site was prepared.

### (4) Preparation of gDNA having a restriction enzyme cleavage site

The desired DNA was treated with EcoRI in a buffer having the following composition (at 37°C for 1 hour), heated after the reaction to inactivate the enzyme and subjected to electrophoresis (agarose gel) to recover gDNA fractions having EcoRI cleavage site.

Composition of the buffer is as follows.

| | |
|---|---|
| • EcoRI | 2 µl |
| • 10 × H buffer | 4 µl |
| • aseptic water | 34 µl |
| Total | 40 µl |

### (5) Restriction enzyme fractions of the substrate DNA were conjugated to the substrate using ligase.

### Industrial Applicability

The csupport for fixing a nucleotide according to the present invention fixes a nucleic acid to be fixed such as DNA using ligase to an oligonucleotide having a restriction enzyme cleavage site and, therefore, as compared with the conventional. method for a direct fixation by means of chemical bond, the nucleic acid such as DNA is able to be fixed in a stable manner.

Further, in accordance with a process for the production according to the present invention, a support for fixing of a nucleotide where nucleic acid such as DNA is able to be stably fixed can be efficiently produced.

When the support for fixing of a nucleotide according to the present invention is put into the market in such a state, a user previously prepares a restriction enzyme cleavage site by cleaving the DNA using the corresponding restriction enzyme whereby the DNA can be fixed easily and stably by the user itself.

## Claims

1. A support for fixing a nucleotide where an oligonucleotide is fixed on a chemically modified substrate, **characterized in that**, the said oligonucleotide has a restriction enzyme cleavage site.

2. The support for fixing of a nucleotide according to claim 1, wherein only one of the strands of the oligonucleotide is bonded onto the chemically modified substrate.

3. The support for fixing a nucleotide according to claim 1 or 2, wherein an oligonucleotide is bonded onto the chemically modified substrate via an amide bond.

4. A process for the production of a support for fixing a nucleotide where a substrate is chemically modified, a single-stranded oligonucleotide is fixed thereto and then another single-stranded oligonucleotide having a complementary base sequence to the above single-stranded oligonucleotide is hybridized to the said above single-stranded oligonucleotide whereupon the oligonucleotide having a restriction enzyme site at the terminal is bonded.

5. The process for the production of a support for fixing a nucleotide according to claim 4, wherein the chemical modification of the substrate comprises chlorination, amination and carboxylation of the substrate surface.

6. The process for the production of a support for fixing a nucleotide according to claim 5, wherein the substrate is chemically modified and then its terminal is activated in the presence of a dehydrating condensing agent.

7. The process for the production of a support for fixing of a nucleotide according to claim 6, wherein the dehydrating condensing agent is carbodiimide and/or N-hydroxysuccinimide.

8. The process for the production of a support for fixing a nucleotide according to claim 4, wherein the chemical modification of the substrate is in such a manner that the substrate surface is chlorinated and aminated and that the formed primary amino group is subjected to dehydration and condensation to one of the ester groups of the activated diester.

9. The process for the production of a support for fixing a nucleotide according to claim 8, wherein the ester group of the activated diester is N-hydroxysuccinimide or p-hydroxysuccinimide.

10. The process for the production of a support for fixing of a nucleotide according to claim 4, wherein the single-stranded oligonucleotide to be fixed to the substrate has 1-10 nucleotide(s) having a primary amine at the terminal of the side to be fixed.

11. The process for the production of a support for fixing a nucleotide according to claim 10, wherein the single-stranded oligonucleotide to be fixed to the substrate has a nucleotide having 1-10 primary amine(s) and then 1-5 thymine or guanine at the terminal of the side to be fixed.

12. The process for the production of a support for fixing a nucleotide according to claim 10 or 11, wherein the single-stranded oligonucleotide to be fixed to the substrate has 1-5 adenine or cytosine and then 1-5 thymine or guanine at the terminal of the side to be fixed.
